Europäisches Patentamt

European Patent Office

Office européen des brevets

(11) Veröffentlichungsnummer: **0 226 041**
A1

(12) # EUROPÄISCHE PATENTANMELDUNG

(21) Anmeldenummer: 86115671.9

(22) Anmeldetag: 12.11.86

(51) Int. Cl.⁴: **A 61 H 23/00**
A 61 B 8/00, G 01 N 29/00

(30) Priorität: 18.12.85 DE 3544710

(43) Veröffentlichungstag der Anmeldung:
24.06.87 Patentblatt 87/26

(84) Benannte Vertragsstaaten:
FR GB IT

(71) Anmelder: DORNIER MEDIZINTECHNIK GMBH
Postfach 1128
D-8034 Germering 1(DE)

(72) Erfinder: Grözinger, Reiner
Greppenstrasse 9
D-8031 Alling(DE)

(72) Erfinder: Isdebski, Kai
Bigenweilerstrasse 27
D-7968 Saulgau(DE)

(72) Erfinder: Wess, Othmar, Dr.
Max-Nadler-Strasse 17
D-8000 München 81(DE)

(72) Erfinder: Windsheimer, Manfred
Ludwigstrasse 2
D-8034 Germering(DE)

(72) Erfinder: Erhardt, Wolfgang
Landsberger Strasse 61
D-8080 Fürstenfeldbruck(DE)

(74) Vertreter: Landsmann, Ralf, Dipl.-Ing.
DORNIER GMBH - Patentabteilung - Kleeweg 3
D-7990 Friedrichshafen 1(DE)

(54) Verfahren und Vorrichtung zur Ankopplung einer Membran an die Haut des menschlichen Körpers.

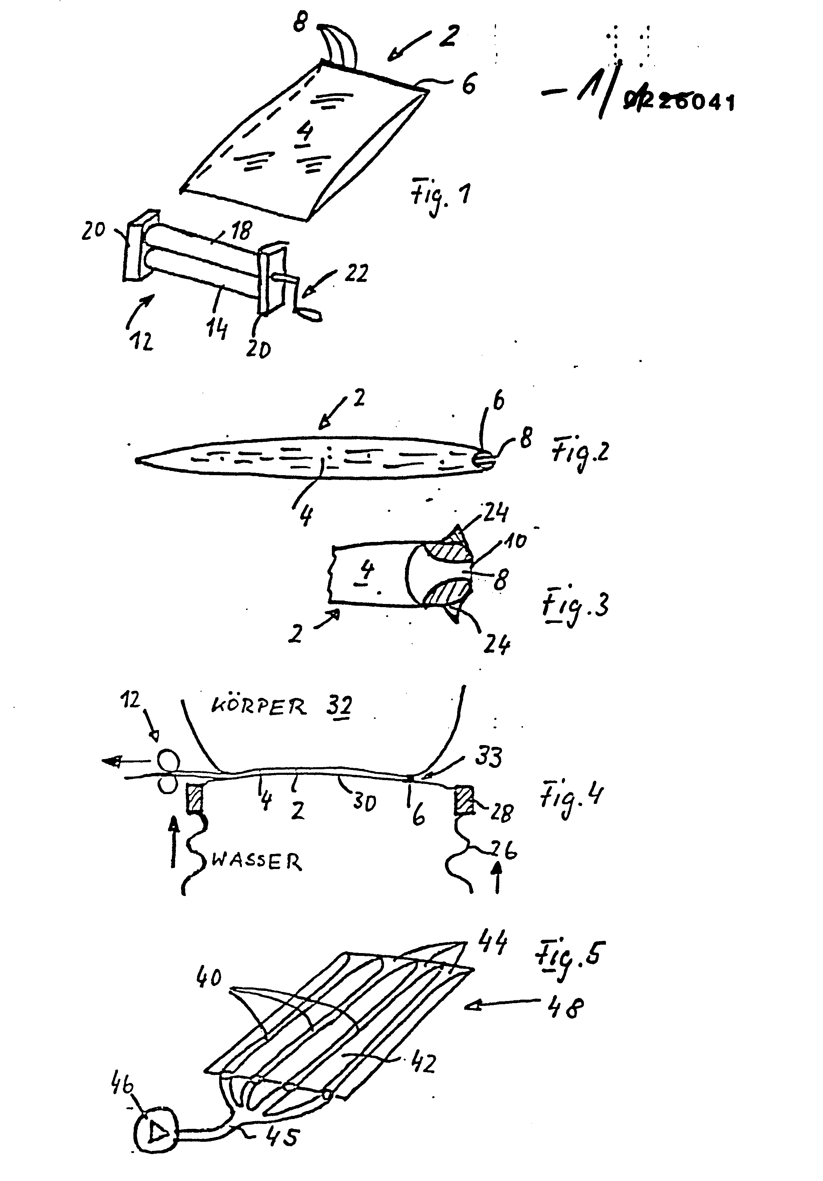

(57) Luftspaltlose Ankopplung eines Körpers an die Membran eines Behandlungsgerät mittels einer Gelschicht, wobei auf die Membran (30) ein Gel abgebendes Bauteil (2) aufgelegt wird, die Membran gegen den Körper (32) gefahren wird und das Bauteil aus dem Spalt (33) zwischen Haut und Membran herausgezogen wird, unter Abgabe von Gel (4) aus einer oder mehreren Öffnungen und vollständiger Füllung des Spalts mit Gel (Figur 4).

Fig. 4

EP 0 226 041 A1

DORNIER MEDIZINTECHNIK GMBH
Postfach 1128
8034 Germering 1

**0226041**

Reg. M 107 EU

Verfahren und Vorrichtung zur Ankopplung einer Membran an die Haut des menschlichen Körpers

Die Erfindung betrifft ein Verfahren und eine Vorrichtung zur Ankopplung einer Membran an die Haut des menschlichen Körpers.

Bei der medizinischen Diagnose und Therapie mittels medizintechnischer Geräte ist ist es häufig notwendig, Strahlung oder mechanische Wellen verlustfrei in den Körper des Patienten einzukoppeln.

Dazu werden Körperhaare durch Rasur entfernt und eine Gelschicht auf den Körper aufgetragen. Wird nun die Membran des Behandlungsgeräts gegen die Gelschicht gedrückt, so kann sich eine luftspaltlose Ankopplung ergeben, jedoch besteht die Gefahr, dass Luftblasen eingeschlossen werden, die den Behandlungserfolg vermindern oder unmöglich machen.

Der Arzt oder medizintechnisches Personal können persönliche handwerkliche Geschicklichkeit bei der Ankopplung von Patienten erwerben, die sich jedoch nicht auf andere Personen übertragen lässt.

Es wurde deshalb z.B. schon vorgeschlagen, den Raum zwischen der Haut des Patienten und der Membran zu evakuieren, wobei jedoch die Gefahr besteht, dass isolierte Lufblasen erhalten bleiben.

Aus den vorgenannten Gründen ist bisher zum Beispiel eine erfolgreiche berührungsfreie Lithotripsie mittels Stosswellen dann möglich, wenn der Patient in einer Wanne mit entgastem Wasser behandelt wird, weil dadurch alle Probleme der Ankoppelung gelöst sind.

Der Erfindung liegt die Aufgabe zugrunde, ein Verfahren und eine Vorrichtung zur Durchführung des Verfahrens zu schaffen, mit deren Hilfe auch ungeübtes Klinikpersonal einen Patienten luftspaltlos an die Membran eines Behandlungsgeräts ankoppeln kann.

Zur Lösung dieser Aufgabe wird ein Gel abgebendes Bauteil (Beutel, Schläuche) zwischen Körperoberfläche und Membran gelegt und aus dem Spalt herausgezogen, wobei Gel unter Überdruck aus einer oder mehreren Öffnungen des Bauteils austritt und den Spalt luftblasenlos auffüllt.

Bei einer Ausführungsform der Erfindung wird die Hautoberfläche des zu behandelnden Patienten, der sich auf einer Patientenliege befindet, mit einem Gel versehen, darauffolgend wird ein gelgefüllter flacher Beutel aufgelegt, das Behandlungsgerät gegen den Körper des Patienten gefahren und der Beutel unter Abstreifen von Luftblasen und Gelabgabe an einer Seitenkante herausgezogen, wobei die Gelabgabe von einer Quetscheinrichtung bewirkt wird.

Weitere Ausgestaltungen der Erfindung sind Gegenstand von Unteransprüchen.

Ausführungsbeispiele der Erfindung werden nachfolgend anhand von Figuren näher erläutert.

Es zeigen:
Fig. 1 einen erfindungsgemässen Gelbeutel,
Fig. 2 eine Schnittansicht von Fig. 1,
Fig. 3 ein vergrössertes Detail von Fig. 2,
Fig. 4 eine prinzipielle Darstellung der Funktionsweise.
Fig. 5 ein weiteres Ausführungsbeispiel der Erfindung

Fig. 1 zeigt einen im wesentlichen rechteckigen Beutel 2 aus einer Kunststoffolie. Im Beutel befindet sich Gel 4 und an einer Stirnseite ist ein Stab 6 eingeschweisst, der mehrere Löcher, Bohrungen oder Schlitze 8 aufweist, aus denen nach Entfernung einer Schutzfolie 10 Gel 4 austreten kann. Dazu dient eine Quetscheinrichtung 12, die ebenfalls in Fig. 1 gezeigt ist und zwei Quetschwalzen 14, 18, ein Gestell 20 sowie eine Kurbel 22 aufweist.

In Figur 2 sind der Gelbeutel 2 und der Stab 6 in Schnittansicht dargestellt, wobei eine Öffnung 8 deutlich zu erkennen ist. In Fig 3 weist der Stab 6 zusätzlich an seiner Unterseite und Oberseite Abstreifer 24 auf, die dazu dienen einen Teil etwa zuvor aufgetragenen Gels abzustreifen und vorhandene Luftblasen herauszutransportieren. Die Öffnung 8 ist dabei strömungsgünstig gestaltet und die Schutzfolie 10 ist zu erkennen.

In Fig. 4 bildet ein Faltenbalg 26 mit einem Rahmen 28 und einer Membran 30 einen geschlossenen wassergefüllten Raum, an dessen anderem Ende sich ein Behandlungsgerät, zum Beispiel ein Stosswellenquelle (nicht gezeigt), befindet. Ein Körper 32, der mit einem Gel versehen sein kann, wird mittels einer nicht gezeigten Patientenliege in Position gebracht und der erfindungsgemässe Beutel 2 wird auf die Membran 30 aufgelegt. Sobald durch Bewegung des Faltenbalgs 31 in Pfeilrichtung sich ein enger Spalt 33 zwischen Körper 32 und Membran 30 mit dazwischen liegendem Beutel 2 gebildet hat, wird der Beutel 2 mittels der Quetscheinrichtung 12 herausgezogen und der Spalt 33 mit Gel 4 gefüllt. Es ergibt sich eine luftspaltlose Ankopplung vom Körper 32 an die Membran 30 des Behandlungsgeräts.

Fig 5 zeigt ein weiteres Ausführungsbeispiel der Erfindung. Hier sind mehrere Schläuche 40 in eine Folie 42 eingeschweisst und die Mündungen 44 der Schläuche so gestaltet, dass sie nahezu die gesamte Stirnseite einnehmen. Die Schläuche 40 kommen von einer Sammelleitung 45 und werden von einer Pumpe 46 mit Gel versorgt. Die Gelpumpe 46 arbeitet dann, wenn die Einrichtung 48 aus dem Spalt 33 zwischen Körper 32 und Membran 30 herausgezogen wird. Auch bei diesem Ausführungsbeispiel können sich im Bereich der Öffnungen 44 ein oder mehrere Abstreifeinrichtungen befinden ( nicht gezeigt).

10.11.86
PaL

DORNIER MEDIZINTECHNIK GMBH
Postfach 1128
8034 Germering 1

— 1 —

0226041

Reg. M 107 EU

Patentansprüche

1. Verfahren zur luftspaltlosen Ankopplung eines Körpers an die Membran eines Behandlungsgeräts mittels einer Gelschicht, dadurch gekennzeichnet, dass auf die Membran (30) ein Gel (4) abgebendes Bauteil (2; 48) aufgelegt wird, dass die Membran (30) gegen den Körper (32) gefahren wird, und dass das Bauteil (2; 48) aus dem Spalt (33) zwischen Haut und Membran heraugezogen wird, wobei Gel (4) aus einer oder mehreren Öffnungen (8; 44) des Bauteils austritt und den Spalt (33) vollständig mit Gel (4) füllt.

2. Vorrichtung zur Durchführung des Verfahrens nach Anspruch 1, gekennzeichnet durch einen Beutel (2), der Gel (4) enthält und an einer Stirnseite einen Stab (6) mit Bohrungen (8) aufweist, aus denen Gel (4) austritt, wenn der Beutel (2) mittels einer Quetscheinrichtung (12) aus dem Spalt (33) zwischen Körper (32) und Membran (30) herausgezogen wird.

3. Vorrichtung nach Anspruch 2, dadurch gekennzeichnet, dass der Stab (6) eine oder mehrere Abstreifeinrichtungen (24) aufweist.

4. Vorrichtung nach Ansprüchen 1 – 3, dadurch gekennzeichnet, dass der Beutel (2) wieder auffüllbar ist.

5. Vorrichtung zur Durchführung des Verfahrens nach Anspruch 1, gekennzeichnet durch mehrere mittels einer Folie (42) miteinander verbundene Schläuche (40), die über eine Sammelleitung (45) mit einer Gelquelle (46) in Verbindung stehen.

10.11.86
PaL

Fig. 1

Fig. 2

Fig. 3

KÖRPER 32

WASSER

Fig. 4

Fig. 5

**0226041**

Nummer der Anmeldung

**EUROPÄISCHER RECHERCHENBERICHT**

Europäisches Patentamt

## EINSCHLÄGIGE DOKUMENTE

EP 86115671.9

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| A | US - A - 2 920 617 (BOIARSKY)<br>* Spalte 2, Anspruch; Fig. 1-4 *<br>-- | 1-5 | A 61 H 23/00<br>A 61 B 8/00<br>G 01 N 29/00 |
| A | US - A - 3 356 086 (BEHNEY)<br>* Spalte 1, Zeilen 44-59; Fig. 1,2 *<br>-- | 1-5 | |
| A | US - A - 4 483 343 (BEER)<br>* Zusammenfassung; Fig. 1,2 *<br>-- | 1-5 | |
| A | CH - A5 - 609 860 (WEHS-GODEL)<br>* Zusammenfassung; Fig. 1-4 *<br>---- | 1-5 | |

**RECHERCHIERTE SACHGEBIETE (Int. Cl 4)**

A 61 H
A 61 B
G 01 N

Der vorliegende Recherchenbericht wurde für alle Patentansprüche erstellt.

| Recherchenort | Abschlußdatum der Recherche | Prüfer |
|---|---|---|
| WIEN | 27-02-1987 | NEGWER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82